# EUROPEAN PATENT SPECIFICATION

(11) **EP 3 086 781 B1**
(45) Date of publication and mention of the grant of the patent: **13.09.2023**
(21) Application number: 14824001.3
(22) Date of filing: 23.12.2014
(51) Int. Cl.: A61K 9/20, A61K 31/00

(54) **PHARMACEUTICAL COMPOSITION OF DPP-IV INHIBITOR IN COMBINATION WITH METFORMIN**
PHARMAZEUTISCHE ZUSAMMENSETZUNG AUS EINEM DPP-IV-HEMMER IN KOMBINATION MIT METFORMIN
COMPOSITION PHARMACEUTIQUE D'UN INHIBITEUR DE LA DPP IV EN COMBINAISON AVEC LA METFORMINE

(30) Priority: 23.12.2013 SI 201300444; 31.07.2014 SI 201400275
(43) Date of publication of application: 02.11.2016
(73) Proprietor: KRKA, d.d., Novo mesto, 8000 Novo mesto (SI)
(72) Inventor: KORASA, Klemen, 8000 Novo Mesto (SI); KUKEC, Simon, 8295 Trzisce (SI); VRECER, Franc, 8351 Straza pri Novem mestu (SI); HUDOVORNIK, Grega, 8000 Novo mesto (SI); SKRABANJA, Vida, 8000 Novo mesto (SI)
(74) Representative: Hoefer & Partner Patentanwälte mbB
(86) International application number: PCT/EP2014/079199
(87) International publication number: WO 2015/097234

(56) References cited:
- WO-A2-2007/041053
- WO-A2-2011/012322
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

### FIELD OF THE INVENTION

The present invention belongs to the field of pharmaceutical technology. Described is a pharmaceutical composition of dipeptidyl peptidase-IV (DPP-IV) inhibitor therapeutic agents and of biguanide therapeutic agents, in particular their combination comprised in single-fixed combination antidiabetic product. More specifically, the present invention relates to a pharmaceutical composition that comprises the active substance vildagliptin or a pharmaceutically acceptable salt of vildagliptin in combination with metformin or a pharmaceutically acceptable salt of metformin. Furthermore, the invention discloses a method of production of the said pharmaceutical composition.

### BACKGROUND OF THE INVENTION

DPP-IV inhibitors work by enhancing the levels of active incretin hormones, which enhance insulin and reduce glucagon secretions, thereby reduce blood glucose levels. They were developed for the treatment or improvement in glycemic control in patients with Type 2 diabetes. Clinical data are available for several selective DPP-4 inhibitors. These agents include alogliptin, anagliptin, dutogliptin, gemigliptin, linagliptin, saxagliptin, sitagliptin, and vildagliptin. Among DPP-IV inhibitors, the most thoroughly studied in clinical trials are vildagliptin and sitagliptin.

Vildagliptin is an active pharmaceutical substance known as (S)-1-[{3-hydroxy-1-adamantyl)amino]acetyl-2-cyano-pyrrolidine and has the structure of formula (I) disclosed in EP 1137635.

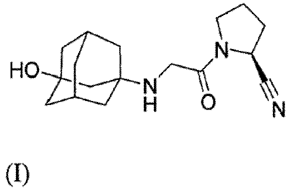

It has been evaluated as monotherapy (marketed as Galvus^{®}) and in combination with other hyperglycemic agents such as metformin (biguanide class), thiazolidinediones, sulfonylureas and insulin.

Metformin with the structure of formula (II) is one of the therapeutic agents, recommended as the first-line antidiabetic medication in most patients with Type 2 diabetes.

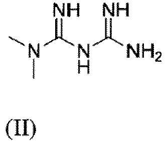

As used herein "metformin" refers to the pharmaceutically active ingredient known under name N,N-dimethylimidodicarbonimidic diamide as well as its pharmaceutically acceptable salts. Metformin is most commonly administered in the form of its hydrochloride salt.

If a glucose control in diabetic patients persists inadequate after the maximum effective and/or tolerated dose of metformin is used, the step-wise addition of other antihyperglycemic agents is recommended. The increasing evidence has shown that combination therapy of DPP-IV inhibitors and metformin provides more effective and safe glycemic control due to complimentary mechanism of action to developed defects in metabolic control of diabetic patients.

WO 2007/041053 (A2) relates to a formulation comprising a dipeptidylpeptidase IV (DPP-IV) inhibitor preferably vildagliptin and metformin, to tablets comprising such formulations and to processes for the preparation thereof. WO 2011/012322 (A2) relates to the synthesis of vildagliptin in the presence of phase transfer catalysts; as well as to the use of vildagliptin or its pharmaceutically acceptable salts for the preparation of solid oral dosage forms.

Most experience related to DPP-IV inhibitor as add-on therapy to metformin which based on clinical studies exists for vildagliptin and sitagliptin.

Combination product of vildagliptin and metformin hydrochloride is marketed under the trademark Eucreas^{®} (50mg/850mg and 50mg/1000mg dosage forms of vildagliptin and metformin hydrochloride). Pharmaceutical formulations of combined respective active ingredients and the process for their preparation are disclosed in EP 1948149. The proprietor assessed moist aqueous granulation, solvent granulation and melt granulation techniques with an intention to properly combine high dose of poorly compactable metformin with moisture sensible vildagliptin. Tablets obtained by moist aqueous granulation were characterized by inadequate physicochemical parameters and were thus unacceptable, while solvent granulation with significant problems (narrow LOD target range and clumping of granulated metformin over time) rendered the granules completely unsuitable for tabletting. Facing with other problems related to processing issues and applicability of wet and/or dry granulation in case of heat and/or moisture sensitive drugs, poor cohesive and compaction properties of combined active ingredients used in described formulation, high drug load, reduced compressibility of added pharmaceutical excipients, such as microcrystalline cellulose, the revealed prior art reference would lead away the skilled person to apply these formulating options as a proper and effective choice.

The proprietor of EP 1948149 came during the opposition procedure to the conclusion that melt granulation was the only formulation technique capable of producing acceptable high load tablets of metformin hydrochloride and vildagliptin (Declaration of J. Lakshman and J. Kowalski of June 4, 2013).

However, melt granulation technique, i.e. hot melt extrusion (HME), has a number of drawbacks. Firstly, this is a novel non-traditional processing technique, which cannot be carried out using traditional manufacturing equipment, so for execution purchase of new high-cost equipment is necessary. In addition, HME process development and optimisation are difficult and complex requiring high process knowledge and consequently time and money consumption for staff education and training. This technique is generally not amenable for scale-down manufacturing that is typically required in early stage development, thus larger quantities of usually expensive active ingredients are used during the development and optimisation of the process. Another point to consider is that process is carried out at elevated temperatures, which make process inappropriate for production of medicinal products containing heat sensitive active ingredients and excipients. High process temperatures also result in high energy consumption. One way to overcome this disadvantage is use of pharmaceutical grade polymers with low melting/softening point, however incorporation of such polymers into the product may lead to physical instability (e. g., phase separation) during handling and storage of agglomerates or final product. Process temperatures are often reduced by addition of plasticizer into the process mixture, but type and amount of plasticizer may affect the dissolution and stability of the product. Besides thermal stability, materials for HME need optimal thermoplastic properties hence number of suitable materials is limited. A further drawback of the method is difficult cleaning of the multicomponent equipment that has to be dissembled before cleaning. Removing remains of material, after manufacturing with high shear forces and elevated temperatures, is time and energy consuming.

Therefore, there remains a need for stable and industrially acceptable pharmaceutical composition comprising vildagliptin and metformin whose manufacturing process is simple, reliable, straightforward, economical and may be conducted using standard manufacturing methods and raw materials.

Surprisingly, it has been found out that stable compositions comprising vildagliptin and metformin hydrochloride can be prepared by wet granulation pharmaceutical process and use of standard pharmaceutical excipients.

### SUMMARY OF THE INVENTION

According to a first aspect, the present invention provides a solid pharmaceutical composition as defined in claim 1.

Furthermore, the present invention provides a process for manufacturing as defined in claim 9, as well as provides a process for manufacturing as defined in claim 10.

The present invention relates to a stable pharmaceutical composition that comprises active substance metformin or a pharmaceutically acceptable salt thereof in combination with DPP-IV inhibitor, which is vildagliptin or a pharmaceutically acceptable salt thereof, which can be produced by a simple, reliable, straightforward, cost-effective process of wet granulation and use of standard pharmaceutical excipients. According to the wet granulation process of the present invention metformin granulate is obtained, to which vildagliptin portion is admixed as in drug particles or granulated form. The pharmaceutical composition of the present invention ensures the free-flowing, physically and chemically stable composition which is easily compressed into fixed oral dosage form, preferably in tablet. Further, compressed tablets have low friability, good compactness, acceptable degree of hardness as well as acceptable dissolution profile and proper disintegration time. The pharmaceutical composition of the present invention enables the use of minimum quantity of excipients in manufacturing of tablets which in spite of high drug load result in acceptable size of final tablets. An increase in compressibility and compactibility properties are provided by appropriate selection and/or amount and/or ratio of binder and diluent in metformin granulate and final composition.

### DETAILED DESCRIPTION OF THE INVENTION

The objective of the present invention is to develop new, stable and industrially acceptable pharmaceutical composition comprising DDP-IV inhibitor, which is preferably vildagliptin, and metformin whose manufacturing process is simple, straightforward, and may be conducted using standard manufacturing methods and raw materials.

Surprisingly, it has been found out that stable compositions comprising DDP-IV inhibitor and metformin can be prepared by wet granulation pharmaceutical process and use of standard pharmaceutical excipients. More specifically, the present invention relates to the stable pharmaceutical composition that comprises vildagliptin or a salt thereof, metformin hydrochloride and standard pharmaceutical excipients.

In the context of the present invention, the term "salt of vildagliptin" may have in particular the meaning of a pharmaceutically acceptable salt of vildagliptin. The term "pharmaceutically acceptable salt" refers in the context of the present application in particular to those salts which are, within the scope of sound medical judgment, suitable for use in contact with the tissues of human beings or animals without excessive toxicity, irritation, allergic response, or other problem or complication, commensurate with a reasonable benefit/risk ratio. A pharmaceutically acceptable salt of vildagliptin can be in particular a hydrochloride, hydrobromide, hydroiodide, hemisulfate, sulfate, lactate, carbonate, 4-acetamidobenzoate, acetate, adipate, alginate, ascorbate, aspartate, benzenesulfonate, benzoate, butyrate, camphorate, camphorsulfonate, cinnamate, citrate, cyclamate, cyclopentanepropionate, decanoate, 2,2-dichloroacetate, digluconate, dodecyl- sulfate, ethane-1,2-disulfonate, ethanesulfonate, formate, fumarate, galactarate, gentisate, glucoheptanoate, gluconate, glucuronate, glutamate, glycerophosphate, glycolate, heptanoate, hexanoate, hippurate, 2-hydroxyethanesulfonate, isobutyrate, laurate, malate, maleate, malonate, mandelate, methanesulfonate, nicotinate, nitrate, octanoate, oleate, orotate, oxalate, 2-oxoglutarate, palmitate, pamoate, pectinate, 3-phenylpropionate, phosphate, L-pyro-glutamate, pivalate, propionate, salicylate, sebacate, hydrogen sebacate, stearate, succinate, tannate, tartrate, hydrogen tartrate, tosylate, or undecanoate salt.

DPP-IV inhibitors such as vildagliptin are sensitive to moisture and are therefore a subject to product stability issues. In order to overcome this applicant has developed such formulation and process that final dosage form shows improved long term stability.

It is an object of the invention to provide a manufacturing process which consistently enables manufacturing formulation comprising DDP-IV inhibitor, most preferably vildagliptin or a salt thereof, and metformin or a salt thereof in a form of free-flowing, stable composition which is easily compressed into a tablet. Further, compressed tablets have low friability, good compactness, acceptable degree of hardness as well as acceptable dissolution profile and proper disintegration time.

It is a further object of the invention to provide high drug load tablet in unit dosage form having such characteristics that it is easily further processed by film coating. The film coating of the invention can optionally have low moisture permeability properties in order to obtain dosage form with high stability of the active ingredients in the tablet.

Further, the invention relates to proper packaging of the final dosage form that is chosen in such a way that moisture and environmental effects are minimized.

In the present invention metformin is formulated into granular product with required mechanical properties such as flowability, compressibility and compactibility by wet granulation (e.g., fluid bed granulation) and vildagliptin is blended with metformin granules after the metformin granulation has been finished. Vildagliptin can be added to metformin in the form of drug particles or in the form of granulate. Due to stability issues vildagliptin granulation can be preferably conducted without solvent or in the presence of non-aqueous solvent.

The present inventors provide a solid pharmaceutical composition comprising metformin in the form of granules and vildagliptin in the form of drug particles or in the form of granules and at least one pharmaceutically acceptable excipient, wherein metformin in the form of granules is prepared by wet granulation. The term "metformin in the form of granules" encompasses metformin or a pharmaceutically acceptable salt thereof in the form of granules. The term "vildagliptin in the form of drug particles or in the form of granules" encompasses vildagliptin or a pharmaceutically acceptable salt thereof in the form of drug particles or in the form of granules.

Furthermore, the solid medicinal preparation of this invention may comprise various ingredients, according to necessity, such as pharmaceutically acceptable lubricants, glidants, binders, emulsifiers, diluents, disintegrants, pH modifiers, solubility enhancers, buffering agents, antioxidant agents, colouring agents, taste and odour improving agents.

The preferred embodiment of the formulation of present invention comprises vildagliptin, metformin hydrochloride, one or more binders, one or more diluents, lubricant and optionally a disintegrant and one or more glidants. Binders are present in final composition in the total amount between 0.5 to 30% by weight of the composition, preferably in the range from 1% to 20%. The terms "formulation", "final composition" and "pharmaceutical composition" can be used interchangeably herein. The solid pharmaceutical composition can comprise a total amount of binder(s) in the range from 0.5 to 30% by weight, preferably in the range from 1% to 20% by weight, based on the weight of the solid pharmaceutical composition, preferably based on the weight of the solid pharmaceutical composition without the weight of optional coating(s).

Diluents can be selected from microcrystalline cellulose, mannitol, sorbitol, lactose, and combinations thereof not specifically listed herein. Preferred diluents are mannitol, lactose, sorbitol and microcrystalline cellulose. Particularly preferred diluents are microcrystalline cellulose and/or mannitol which provide greater tablet hardness and appropriate final product stability. Most preferably, microcrystalline cellulose and/or mannitol grades with high compactability and compressibility are used, i.e. Ceolus^{™} KG or LTF grades for microcrystalline cellulose and Parteck^{®} M200 or M100 grade for mannitol. These grades offer required hardness and friability of high drug load tablets. Preferred amount of diluent or diluent mixture, wherein the diluent is preferably microcrystalline cellulose and/or mannitol, in the invention in particular in the pharmaceutical composition according to the present invention is between 0.5% and 25% by weight of the composition. Particularly preferred range is from 1% to 15% by weight. Further diluents can be admixed with microcrystalline cellulose and/or mannitol in the composition of the present invention. In such case the preferred total amount of all diluents in the present invention in particular in the pharmaceutical composition according to the present invention is between 1% and 30% and most preferred between 1% and 20% by weight.

The applicant has found that optimal processability, final dosage form properties and adequate stability are achieved if composition of the present invention includes both binder and diluent. Presence of these two pharmaceutical excipients allows smooth execution of granulation process, good flow properties and compression mixture with enhanced compressibility and compactibility. Especially compressibility and compactibility of formulation containing both diluent and binder, rather than just binder, are improved. Further, the applicant has found that above mentioned advantages are in particular achieved in case when diluent and binder are present in the appropriate weight ratio. According to the invention the weight ratio between diluent and binder is from 2.5:1 to 1:5, preferably from 2:1 to 1:4, even more preferably from 1.5:1 to 1:3. Preferred diluents according to the present invention are mannitol, lactose, sorbitol and microcrystalline cellulose and combinations thereof. Particularly preferred diluents are microcrystalline cellulose and/or mannitol.

Preferred binders are hydroxypropyl cellulose (HPC), hydroxypropyl methylcellulose (HPMC), and hydroxyethyl cellulose (HEC) and combinations thereof. Hydroxypropyl cellulose is particularly preferred. Most preferably a combination of microcrystalline cellulose and/or mannitol with hydroxypropyl cellulose is used.

The lubricant can be selected from stearic acid, a metal salt of stearic acid such as magnesium stearate, talc, colloidal silica, a wax variety such as beads wax, spermaceti, boric acid, adipic acid, sodium sulphate, fumaric acid, stearyl sodium fumarate, sucrose aliphatic acid ester, a lauryl sulphate such as sodium lauryl sulphate, magnesium lauryl sulphate, starch derivative such as corn starch, potato starch, glyceryl behenate, behenoyl polyoxyl glyceride etc. Preferred lubricants are metal salt of stearic acid such as magnesium stearate, stearic acid, sodium stearyl fumarate or their mixture, preferably magnesium stearate. Lubricants are present in an amount from about 0.1% to 5% by weight of the composition, preferably from 0.25% to 3%.

Optionally one or more glidants can be added to the formulation of the invention. Examples of pharmaceutically accepted glidants are talc, colloidal silica, hydrophobic colloidal silica, magnesium oxide, powdered cellulose, silicates such as magnesium silicate, magnesium trisilicate etc. Glidant may be present in the amount from 0.1% to 5% by weight of the composition.

Optionally, disintegrants can be part of the formulation. Different types of disintegrants such as crospovidone, croscarmellose sodium, carmellose sodium or calcium, low substituted hydroxypropyl cellulose, sodium starch glycolate, salts of carboxymethyl cellulose, salts of polacrilin and others can be used in the invention. The disintegrants can be added to the formulation in the granulation phase of one or both active substances, in the preparation of the compression mixture, i.e. extragranularly, or in both phases.

Two active ingredients are present in composition of the invention, i.e. DPP-IV inhibitor, which is vildagliptin or a pharmaceutically acceptable salt thereof, and metformin. In the context of the present invention most preferably vildagliptin is used and formulated with metformin into the combined solid pharmaceutical composition. Metformin might be present in different chemical entities such as salts, hydrates, solvates, polymorphs and/or cocrystals. For example metformin salts are disclosed in WO 99/29314 and WO 2009/144527. Most preferably metformin salt is selected from metformin hydrochloride.

The amount of vildagliptin or acceptable salt thereof is between 1% and 30% by weight of the total amount of solid medical preparation (the terms "medical preparation" and "pharmaceutical composition" being used interchangeably herein), preferably from 1.5% to 15% by weight. The solid pharmaceutical composition of the present invention can comprise vildagliptin or acceptable salt thereof in an amount between 1% and 30% by weight, preferably from 1.5% to 15% by weight, based on the weight of the solid pharmaceutical composition.

The amount of metformin or acceptable salt thereof is between 60% and 98% by weight of the total amount of solid medical preparation (the terms "medical preparation" and "pharmaceutical composition" being used interchangeably herein), preferably between 70% and 95% by weight, more preferably between 70 and 80% by weight of the composition. The solid pharmaceutical composition of the present invention can comprise metformin or acceptable salt thereof in an amount between 60% and 98% by weight, preferably between 70% and 95% by weight, more preferably between 70 and 80% by weight, based on the weight of the solid pharmaceutical composition.

Metformin is present in the composition in the form of granules which comprises a drug and both a binder and a diluent and optionally other excipients selected from disintegrants, glidants and/or surface active agents. Metformin hydrochloride granules can be produced by state of the art granulation techniques, where wet granulation is preferred. Wet granulation of metformin hydrochloride is executed using at least one solvent selected from organic solvents such as alcohols (e.g., methanol, ethanol, isopropanol), ketones (e.g., acetone) or esters (e.g., ethylacetate); water or mixtures of different solvents including mixtures of water and organic solvents, where water is being preferred solvent or dispersion medium. Specially preferred is aqueous wet granulation process of metformin hydrochloride with at least one pharmaceutically acceptable excipient.

Binders used in manufacturing of metformin hydrochloride granulate can be selected from hydroxypropyl cellulose, hydroxypropyl methyl cellulose, hydroxyethyl cellulose. Preferably binders are selected from hydroxypropyl cellulose (hyprolose, HPC), hydroxypropyl methylcellulose (hypromellose, HPMC), and hydroxyethyl cellulose (HEC) or mixture thereof. Binders in metformin granulate are present in the amount between 0.5% to 20% by weight of the final composition, preferably between 1% and 15% by weight. The solid pharmaceutical composition of the present invention can comprise metformin granulate, which metformin granulate can comprise binder(s) in an amount between 0.5% to 20% by weight, preferably between 1% and 15% by weight, based on the weight of the solid pharmaceutical composition.

The terms metformin in the form of granules and metformin granulate can be used interchangeably herein. The terms granule and granulate can be used interchangeably herein. Metformin in the form of granules can be in particular granules comprising or consisting of metformin or a pharmaceutically acceptable salt thereof, preferably comprising metformin or a pharmaceutically acceptable salt thereof. Metformin granulate can be in particular granulate comprising or consisting of metformin or a pharmaceutically acceptable salt thereof, preferably comprising metformin or a pharmaceutically acceptable salt thereof.

Metformin granulate comprises a drug and both a binder and a diluent. The applicant found that in the process of wet granulation addition of diluent to the composition increases compressibility of the formulation and hardness of tablets significantly so it is preferred to granulate metformin in particulate form together with binder and one or more diluents. The preferred diluents in the metformin granulate are microcrystalline cellulose and mannitol. Total amount of diluents in the metformin granulate is in the range from 0.5% to 20% by weight, preferably from 1% to 15% by weight of final composition. The preferred binder in the metformin granulate is selected from hydroxypropyl cellulose, hydroxypropylmethyl cellulose and hydroxyethyl cellulose. Hydroxypropyl cellulose is particularly preferred. Total amount of binder in the metformin granulate is in the range from 0.5% to 20% by weight, preferably from 1% to 15% by weight of final composition. Further excipients such as disintegrants can optionally be incorporated into the metformin containing granulate. The solid pharmaceutical composition of the present invention can comprise metformin in the form of granules (metformin granulate), which metformin in the form of granules (metformin granulate) can comprise a total amount of diluent(s) in the range from 0.5% to 20% by weight, preferably from 1% to 15% by weight, based on the weight of the solid pharmaceutical composition and/or which metformin in the form of granules (metformin granulate) can comprise a total amount of binder(s) in the range from 0.5% to 20% by weight, preferably from 1% to 15% by weight, based on the weight of the solid pharmaceutical composition .

Vildagliptin can be in one aspect of the invention incorporated into solid composition by direct mixing of vildagliptin drug particles with metformin containing granulate together with at least one further excipient selected from glidants, lubricants, disintegrants, stabilisers and/or pH modifiers. In another aspect of the present invention, vildagliptin can be formulated into solid composition in the form of granulate, where both granulates, i.e. vildagliptin or its salt and metformin hydrochloride containing granulates, are mixed together with at least one further excipient. When vildagliptin is admixed in the form of granulate the latter contains at least one pharmaceutically acceptable excipient selected from diluent, binder, disintegrant, glidant or lubricant. Preferably at least one excipient selected from diluent and binder is used in the formulation of vildagliptin granulate. Vildagliptin granulate is manufactured either by wet granulation, dry granulation or melt granulation.

Addition of DDP-IV inhibitor, which is preferably vildagliptin, in the form of granules into compression mixture, has positive influence on processability and final drug properties. Main advantage of admixing vildagliptin in granulate form are enhanced compressibility and compactibility of the compression mixture, which enables production of tablets with high and consistent hardness. Secondly, blending of vildagliptin with metformin granulate and pharmaceutical excipients is improved due to smaller particle size difference between the two granulates. This enables production of homogeneous compression mixture and required content uniformity of both active ingredients is achieved. Granulation of both active ingredients separately also reduces contact surface between them in the final drug product. Smaller contact surface reduces chemical degradation rate and is responsible for better product stability. The applicant has also found that tablets prepared in such a manner have higher and less variable dissolution profiles, which was attributed to higher tablet porosity and/or more homogeneous tablet hardness.

The dry granulation method is a method that can be performed by process known in the art as slugging or roller compaction. DPP-IV inhibitor, which is preferably vildagliptin, optionally sieved, is mixed with at least one excipient and compressed into a compact/comprimate by using roller compactor or compression machine. The obtained compact/comprimate is crushed and optionally sieved to appropriate particle size distribution. Vildagliptin granulate manufactured by dry granulation process comprises active pharmaceutical ingredient and at least one pharmaceutically acceptable excipient. Preferably, pharmaceutical excipient built in the granulate is binder, diluent or combination of binder and diluent. Optionally, additional excipients (e.g., disintegrant) can be incorporated into the granulate. Total amount of binder, diluent or mixture thereof is from 10 to 80 %, preferably from 20 to 60 % by weight of vildagliptin granulate. The terms vildagliptin in the form of granules and vildagliptin granulate can be used interchangeably herein. Vildagliptin in the form of granules can be in particular granules comprising or consisting of vildagliptin or a pharmaceutically acceptable salt thereof, preferably comprising vildagliptin or a pharmaceutically acceptable salt thereof. Vildagliptin granulate can be in particular granulate comprising or consisting of vildagliptin or a pharmaceutically acceptable salt thereof, preferably comprising vildagliptin or a pharmaceutically acceptable salt thereof. Vildagliptin in the form of drug particles can be in particular vildagliptin particles (vildagliptin in particle form).

In an embodiment, the solid pharmaceutical composition of the present invention comprises first granules comprising metformin or a pharmaceutically acceptable salt thereof and second granules comprising vildagliptin or a pharmaceutically acceptable salt thereof. These first granules can have a composition different from the composition of the second granules. Preferably, the first granules are free from vildagliptin or a pharmaceutically acceptable salt thereof. Preferably, the second granules are free from metformin or a pharmaceutically acceptable salt thereof.

Wet granulation of DPP-IV inhibitor, which is preferably vildagliptin or its pharmaceutically acceptable salt, is performed by the state of the art granulation processes and equipment such as low shear, high shear or fluid bed granulator, where granulation liquid is based on water, one or more acceptable organic solvents or mixture thereof. Preferably granulation liquid for vildagliptin granulation is based on non-aqueous solvent/vehicle. Most preferably solvent/vehicle for vildagliptin wet granulation is selected from alcohols such as methanol, ethanol, isopropanol or ketones such as acetone. In case of using alcohols as solvents/vehicles, they can be used in absolute form (pure alcohol) or as a mixture with water, where the alcohol concentration is at least 70 V/V%. Granulation liquid can be in one aspect of the invention pure solvent or in an another aspect can be a dispersion, where excipients selected from binders, diluents, pH modifiers, stabilisers, antioxidants and/or surfactants are dispersed meaning dissolved, suspended, or emulsified in the solvent. Vildagliptin granulate manufactured by wet granulation comprises a drug and at least one pharmaceutically acceptable excipient. Preferably, vildagliptin granules comprise active substance, binder and diluent. Optionally, disintegrant is admixed. Preferred binders in the vildagliptin granulate produced by wet granulation are hydroxypropyl cellulose (HPC), hydroxypropyl methylcellulose (HPMC), and hydroxyethyl cellulose (HEC). Hydroxypropyl cellulose is particularly preferred. Preferred diluents are mannitol, lactose, sorbitol and microcrystalline cellulose. Total amount of binder is between 1 and 20 %, preferably between 1 and 15 % by weight of vildagliptin granulate. Preferred amount of diluent is between 10 and 80 %, even more preferred between 20 and 60 % by weight of vildagliptin granulate. Vildagliptin in the form of granules (vildagliptin granulate) can comprise diluent in an amount between 10 and 80 % by weight, preferably between 20 and 60 % by weight, based on the weight of the vildagliptin in the form of granules (vildagliptin granulate), and/or one or more binders in a total amount of binder(s) between 1 and 20 % by weight, preferably between 1 and 15 % by weight, based on the weight of the vildagliptin in the form of granules (vildagliptin granulate). Using ethanol as a solvent and fluid bed granulating techniques are the preferred choice to manufacture vildagliptin granulate with optimal characteristics and compatibility with the metformin granulate of the present invention. Preferably ethanol aqueous solution with concentration of ethanol 70 V/V % or higher is used, e.g. 70 V/V %, 96 V/V %, anhydrous ethanol.

Melt granulation of DPP-IV inhibitor, which is preferably vildagliptin, can be performed by the process of the hot melt agglomeration or hot melt extrusion. Hot melt agglomeration can be performed by agglomeration of active ingredient, binder and optionally admixed excipients in one aspect by so called melt in process, where mixture of drug and excipients including binder is heated up to a temperature of binder melting or softening point (in situ agglomeration). In another aspect of the present invention melted binder is sprayed onto the powder mixture - so called spray on agglomeration. Optionally further excipients can be dispersed in molten binder. The granulation temperature can be optionally increased up to 15, preferably 10 °C above the melting/softening point of the binder in order to achieve better spreading of molten binder across the powder mixture. Binders suitable for melt agglomeration can be selected from excipients having melting or softening point below 150 °C, preferably below 130 °C and most preferably below 100 °C such as polyethylene glycols, polyoxyglycerides, poloxamers, esters of polyethylene glycols, sucrose esters, fats (e.g., glyceryl behenate, glyceryl palmitate, glyceryl monostearate), waxes (beewax, carnauba wax), cetostearyl alcohol, stearic acid, and hydrogenated castor or cottonseed oil. Hot melt extrusion can be performed by the state of the art process and equipment where the active ingredient is mixed with polymer and optionally other excipients. The mixture is afterwards heated in the extruder to approximate melting or softening point of the polymer. Polymers selected for hot melt extrusion have melting or softening temperature below 170 °C. Plasticizers such as polyethylene glycol with average molecular weight in the range of 200 to 8,000, preferably 350 to 6,000, propylene glycol, triethyl citrate, tributyl citrate, triacetin, dibutyl sebacate, diethyl phthalate and/or glycerol monostearate can be used to reduce the softening point of polymers used for hot melt extrusion to below 150 °C. The weight ratio of polymer and plasticizer (polymer : plasticizer) for hot melt extrusion is from 20 to 1 to 3 to 1, preferably 10 weight % concentration of plasticizer calculated on to the weight of polymer is used.

In special embodiment of the present invention granules containing vildagliptin or its salt can be coated with a water soluble film coating by the state of the art coating process and equipment. Fluid bed coating process is preferred, where bottom spray fluid bed process and equipment such as but not limited to Glatt GPCG, Hüttlin diskjet or HDGC systems, Innoyet ventilus, Aeromatic Precision-coater and the like are mostly preferred. Film coating of vildagliptin granules is performed by coating dispersion which in one embodiment of present invention is based on dispersion of suitable polymer and optionally other excipients selected from antitacking agents, pigments and colorants, plasticizers and a solvent. Solvents can be selected from alcohols (such as methanol, ethanol, isopropanol), ketones (such as acetone, methylethyl ketone), esters (such as ethylacetate), water or mixtures thereof. In case of using mixtures of organic solvent and water, such mixture contains less than 50 vol% of water. Applied film coating can optionally have decreased permeability for gases. Polymers, which are basic constituent of the film coating, can be selected from water soluble polymers such as cellulose ethers such as hyprolose and/or hypromellose, polyvinyl alcohol and its derivatives such as block copolymers of polyvinyl alcohol and polyethylene glycol, commercially available as Kollicoat^{®} IR and/or Kollicoat^{®} Protect, derivatives of polymethacrylic acid such as Eudragit^{®} E, carboxymethyl cellulose or the like. Antitacking agents can be selected from talc, metal salts of fatty acids such as calcium or magnesium stearate, glycerol monostearate, stearic acid or the like. Plasticizers can be selected from polyethylene glycol having average molecular weight in the range 300 to 10,000, propylene glycol, triethyl citrate, tributyl citrate, triacetin, dibutyl sebacate, diethyl phthalate and/or glycerol monostearate or the like. Pigments can be selected from metal oxydes such as iron and/or titanium oxydes. Colorants can be selected from water soluble pharmaceutically acceptable colours which are proven to be nontoxic for humans.

The advantage of applying of film coating onto vildagliptin granules is beside improved chemical stability of the product also improved processability of granulate due to their higher smoothness and decreased contact of vildagliptin with metformin in the final solid dosage form.

Compression mixture of the present invention comprises metformin granulate, DDP-IV inhibitor, which is preferably vildagliptin, in the form of drug particles or in form of granulate and optionally one or more pharmaceutically acceptable excipients. In preferred embodiment of the invention besides of metformin granulate and vildagliptin granulate/particles at least one lubricant is admixed into the compression mixture. Other pharmaceutically acceptable excipients such as diluents, glidants, disintegrants, binders, colouring agents, solubility enhancers, stabilisers can optionally be admixed in order to achieve desired characteristics of compression mixture and final dosage form.

The amount of the lubricant in the compression mixture can be in the range of 0.1% to 5% by weight, preferably between 0.25% and 3% by weight. Preferred lubricants in the formulation are selected from metal salts of stearic acid, stearic acid or sodium stearyl fumarate. The solid pharmaceutical composition of the present invention can comprise lubricant in an amount in the range of 0.1% to 5% by weight, preferably between 0.25% and 3% by weight, based on the weight of the solid pharmaceutical composition, preferably based on the weight of the solid pharmaceutical composition without the weight of (optional) coating(s).

It is preferred that after tablet-compression process tablets are further film-coated. Application of film coating may bring many benefits to the existing dosage form, e.g. controlled drug release, better appearance, easier swallowing, protection from environmental factors such as oxygen and/or moisture, taste masking, better mechanical properties of tablets, protection from gastric acid and other endogenous substances etc. Film coating is present in the range from 1% to 20% by weight of the total amount of solid medical preparation, preferably between 1% and 10% by weight. Preferred polymers used for film coating are cellulose ethers such as hyprolose (hydroxypropyl cellulose; HPC), hypromellose (hydroxypropyl methylcellulose; HPMC), carboxymethyl cellulose; polyvinyl alcohol or block copolymer of polyvinyl alcohol and polyethylene glycol (e.g., Kollicoat^{®} IR and Kollicoat^{®} Protect), derivatives of polymethacrylates such as Eudragit^{®} E, and other similar film coatings having decreased permeability for moisture and/or oxygen. Further, additives selected from pharmaceutically acceptable plasticizers, colorants, pigments and antitacking agents are included in the coating in order to achieve optimal coating characteristics. Examples of plasticizers include propylene glycol, polyethylene glycol, polypropylene glycol, glycerol, sorbitol, glycerol triacetate, diethyl phthalate, lauric acid, sucrose, dextrose, sorbitol, triacetin, acetyl triethyl citrate, triethyl citrate, tributyl citrate and acetyl tributyl citrate, etc.

Pigments can be selected from metal oxides such as titanium dioxide, iron oxide, iron sesquioxide, lakes such as aluminium lake, talc, etc. or mixture thereof.

Examples of antitacking agents that might be used are talc, colloidal silica, magnesium oxide, glycerol monostearate, etc.

Film coatings based on polyvinyl alcohol (PVA) and block copolymer of polyvinyl alcohol and polyethylene glycol (e.g., Kollicoat^{®} IR and Kollicoat^{®} Protect) show the most optimal barrier function.

Final dosage form is contained in an appropriate packaging material in order to minimize moisture and environmental effects on chemical and physical stability of the product. It is preferred that moisture permeability of primary packaging is decreased and consequently moisture sorption is diminished. Low moisture permeable primary packaging materials such as aluminium or polychloro-3-fluoroethylene homopolymer/PVC laminate can be used with the thickness in the range from 10 to 40 µm in case of Al/Al blisters and 10 to 110 µm in case of Al-polychloro-3-fluoroethylene homopolymer/PVC laminate blisters. Optionally, dosage forms containing vildagliptin or pharmaceutical acceptable salt thereof and metformin or pharmaceutical salt thereof can be packed into primary packaging with desiccant. Desiccant can be incorporated into the primary packaging together with final product or it is integrated in the wall or closure system of the primary packaging. To reduce environmental oxygen exposure, packaging can be carried out under reduced pressure or in an inert gas atmosphere. Interaction between final dosage form and oxygen can be also reduced by the use of oxygen absorbers. Preferably, packaging with a desiccant is used.

In the development of the present formulation the applicant has found that metformin particle size has effect on bulk volume, compressibility, compactibility and flow properties of the granulate. It is particularly advantageous that average particle size of metformin or acceptable salt thereof is between 20 to 240 µm, in particular between 40 to 240 µm, especially between 40 to 200 µm, preferably between 60 and 180 µm.

Metformin particles with average particle size smaller than 40 µm, especially smaller than 20 µm result in high cohesive forces, which hinder normal course of the process. Metformin particles with average particle size higher than 240 µm result in a metformin granulate with poor compressibility and compactibility which disable production of tablet cores with advantageous degrees of hardness, friability and resistance to chipping.

Vildagliptin particle size and particle size distribution has also an effect on processibility, i.e. compressibility and compactibility of the compression mixture and on the performance of blending process. The preferred average particle size of vildagliptin or acceptable salt thereof is between 10 and 250 µm, preferably between 20 and 150 µm.

It has been discovered that particle size in the above mentioned range enables good blending performance and proper compressibility and compactibility when vildagliptin is admixed into compression mixture in the form of particles. When vildagliptin particles are outside this size range difficulties during blending might occur, e.g. longer blending times, poor blend homogeneity, clusters of vildagliptin due to particle agglomeration. It has been also discovered that this particle size distribution enables production of tablet cores with acceptable physical characteristics, i.e. hardness, friability, porosity.

Furthermore, it has been found that above defined vildagliptin particle size range enables optimum feasibility also in case when vildagliptin is admixed into the compression mixture in the form of granules. Selected particle size distribution makes granulation process feasible and enables production of granulate with satisfactory bulk volume, flow properties, blending characteristics and ability to form compressible and compactible compression mixture.

The average particle size was determined by laser diffraction method using Malvern Mastersizer MS2000 equipped with Hydro 2000S dispersion unit in case of metformin and vildagliptin. In case of metformin isopar was used as dispersant, in case of vildagliptin light liquid paraffin, in particular with the viscosity of 25-80 mPas was used. The term average particle size as used herein refers to the volume mean particle diameter.

The process of the invention is carried out in the following order:
i) production of metformin granulate,
ii) optionally production of vildagliptin granulate,
iii) blending of vildagliptin in form of drug particles or granulate and optionally other pharmaceutically acceptable excipients with metformin granulate,
iv) optionally blending lubricant with the mixture obtained in step iii) to obtain compression mixture,
v) compressing the resulting compression mixture to form tablets, preferably tablets in unit dosage form,
vi) optionally film coating of tablets obtained in step v).

The granulation of step i) is wet granulation, most preferably the granulation of step i) is fluid bed granulation.

The bulk volume of the granulation manufactured in point i) showed correlation with ability to achieve adequate compressibility and compactibility of the compression mixture. It was found that optimal bulk volume of the metfomin granulate is in the range from 1.5 mL/g to 3.2 mL/g, preferably in between 1.8 mL/g and 3.0 mL/g. It was also found if bulk volume measurement is too high (i.e., >3.2 mL/g) production of tablets of acceptable size is difficult.

The final moisture level of granulate manufactured in point i) also showed to be critical. If loss on drying value (LOD) is too high the stability of final drug product is reduced. On the other hand, if LOD is too low the compactibility of compression mixture is reduced. The optimal LOD is in the range of 0.025% to 1.5 %, preferably of 0.05% to 0.8%.

Preferably the granulation of step ii) is wet, dry or melt granulation, most preferably wet granulation is used.

It was found that after sieving through a 20 mesh (0.84 mm openings) at least 40%, preferably at least 60%, of granules produced in point i) are in the particle size range from 125 to 500 µm. This particle size distribution enables good flow properties and appropriate compression and compaction properties of the compression mixture.

At least 40%, more preferably at least 50%, of the granules produced in point ii) are in the particle size range from 71 to 250 µm. The bulk volume of granulate is in the range from 0.5 mL/g to 3.0 mL/g, preferably in the range from 1.0 mL/g to 2.5 mL/g. Such particle size distribution and bulk volume provide acceptable physical properties of the final compression mixture and acceptable blending with metformin granulate and optionally added excipients.

The optimal LOD value of granulate obtained in point ii) is in the range of 0.05% to 1.8%, preferably from 0.1% to 0.9%, in order to achieve final drug stability and feasibility of further manufacturing process.

Particle size distribution, bulk volume and LOD value of the compression mixture obtained in step iv) are critical for achieving appropriate physical properties and stability of the final product. Blend properties depend on metformin granulate characteristics, vildagliptin particles or vildagliptin granulate characteristics, characteristics of optionally added excipients, and on blending process itself.

The optimal LOD of the compression mixture is in the range of 0.05% to 1.7%, preferably of 0.1% to 0.8%. Preferred bulk volume of the blend is approximately 2.5 mL/g, preferably a range of 1.5 mL/g to 3.2 mL/g, more preferably of 1.8 mL/g to 3.0 mL/g. It is further preferred that at least 40%, preferably at least 50%, of the compression mixture is in the particle size range from 125 to 500 µm.

LOD measurements were done on a 3-4 g sample using Mettler Toledo halogen moisture analyzer set at 90 °C. When sample reached constant mass the end point was determined.

Particle size measurements of both granulates and compression mixture were conducted using air jet sieve particle size analyzer (Hosokawa Alpine 200 LS). Sieves with mesh size 71, 125, 250, 500 and 710 µm were used to carry out the analysis.

Bulk volume was measured using Method 1 (Measurement in a Graduated Cylinder) as prescribed in European Pharmacopoeia 8.0, chapter 2.9.34. Bulk Density and Tapped Density of Powders, paragraph Bulk density. Bulk volume was calculated by dividing measured volume with the weight of the applied material.

Tapped volume was measured using Method 1 as prescribed in European Pharmacopoeia 8.0, chaper 2.9.34. Bulk Density and Tapped Density of Powders, paragraph Tapped density. Volume was read after 1250 taps had been applied. Tapped volume was calculated by dividing measured volume with the weight of the applied material.

During development of the invention the applicant has found that flow properties of the compression mixture (blend from point iv)) can be described well by use of Hausner ratio (ratio between bulk and tapped volume). It is preferred that Hausner ratio is in the range of 1.0 to 1.4.

The compression of compression mixture from step iv) is carried out at a compression force of 5 kN to 60 kN depending of the compression blend characteristics and dimensions of the punch. The hardness of the tablet obtained in step v) is between 50 and 400 N, friability of the tablet is lower than 0.5%, tablet thickness is comprised between 4 and 9 mm. Tablet dimensions and shape are selected in such a way that they do not cause difficulties in swallowing at patients, so tablet's length is less than 22 mm and width is less than 12 mm. The target disintegration time is between 10 min and 30 min.

Tablet hardness was measured using Erweka Multicheck machine, every time 20 tablets were tested and average tablet hardness was calculated.

Friability was determined on 10 tablets which were subjected to 10 minutes testing at 40 rpm using Erweka friability tester. The test was carried out in accordance with European Pharmacopoeia.

Disintegration testing was carried out in apparatus A (European Pharmacopoeia) using water at a temperature of 37 °C as disintegration medium. The test was carried out in accordance with European Pharmacopoeia.

Tablets from step v) can be further film coated. Film coating dispersion can be prepared using different solvents, like water or organic solvents such as alcohols (e.g., methanol, ethanol, isopropanol), ketones (e.g., acetone), and mixtures thereof. The preferred solvent is water. Preferred composition of coating suspension (calculated as dry material) comprises 1-99% by weight preferably 1-95% of polymer, 1-50% by weight preferably 1-40% of plasticizer , 0.1-20% by weight preferably 0.1-10% of colorant/pigment and 0.1-20% by weight preferably 1-10% of antitacking agent. The following polymers: cellulose ethers (e.g., HPMC), polyvinyl alcohol or block copolymer of polyvinyl alcohol and polyethylene glycol are particularly preferred. The thickness of the coating is in the range of 2-40 µm, preferably 3-30 µm, most preferably 3-25 µm, measured on the cross-section of film-coated tablet using scanning electron microscope. Hardness of film coated tablets obtained in step vi) is between 100 and 500 N, tablet thickness is between 4.5 mm and 9.5. Tablet's length is less than 25 mm and width less than 14 mm. The target disintegration time is between 10 and 40 min.

LOD values of coated tablets produced in step vi) are between 0.1% and 1.5%, preferably between 0.2% and 0.9%. Low moisture content in the final dosage form has shown to be crucial in achieving appropriate level of chemical stability.

The present invention can be illustrated in one of its embodiments by the following nonlimiting examples.

All quantities of raw materials in composition for each example are given in amounts for one tablet.

### EXAMPLES 1-10

Further examples present manufacturing process where vildagliptin in the form of particles is blended with metformin granules.

### Preparation of metformin granulate

**Table 1 -composition of metformin granulate: examples 1a-10a (quantities are calculated in amount for one tablet):**

| **Granulate** | | | | | |
|---|---|---|---|---|---|
| Example | 1a | 2a | 3a | 4a | 5a |

| **Components (mg)** | | | | | |
|---|---|---|---|---|---|
| Metformin hydrochloride | 1000 | 1000 | 1000 | 1000 | 1000 |
| Microcrystalline cellulose | 30 | 44 | - | 34 | 34 |
| Lactose monohydrate | 14 | - | - | - | - |
| Mannitol | - | - | - | - | - |
| Low substituted HPC | - | - | - | - | - |
| HPC | 55 | 55 | 53 | - | - |
| HEC | - | - | - | - | 55 |
| HPMC | - | - | - | 55 | - |
| Purified water | q.s | q.s. | q.s. | q.s. | q.s. |

**Table 1 (continued):**

| **Granulate** | | | | | |
|---|---|---|---|---|---|
| Example | 6a | 7a | 8a | 9a | 10a |

| **Components (mg)** | | | | | |
|---|---|---|---|---|---|
| Metformin hydrochloride | 1000 | 1000 | 1000 | 1000 | 1000 |
| Microcrystalline cellulose | - | - | 70 | 69 | - |
| Lactose monohydrate | - | 30 | - | - | 10 |
| Mannitol | 20 | 35 | - | - | - |
| Low substituted HPC | 10 | 24 | 14 | - | - |
| HPC | 49 | 60 | - | 60 | 55 |
| HEC | - | - | - | - | - |
| HPMC | - | - | 65 | - | - |
| Purified water | q.s. | q.s. | q.s. | q.s. | q.s. |

The solid components shown in Table 1 (without binder) were homogenized in a fluid bed granulator. Afterwards, granulation liquid prepared by dissolving a binder (HPC, HEC or HPMC) in the purified water was sprayed onto the powder mixture. Alternatively, a part or whole amount of binder can be put into the powder mixture and purified water is sprayed over this mixture. Granulate mixture temperature during the spraying was held in the range from 25 °C to 40 °C. The obtained granulate was dried in fluid bed dryer at a specified temperature. The drying was stopped when granulate temperature reached 40 to 50 °C.

### Preparation of compression mixtures

The components shown in Table 2 were mixed in the container mixer to obtain final compression mixture (Examples 1-10).

**Table 2 - components added to the metformin granulates (quantities are calculated in amount for one tablet):**

| **Compression mixture** | | | | | |
|---|---|---|---|---|---|
| Example | 1 | 2 | 3 | 4 | 5 |

| **Components (mg)** | | | | | |
|---|---|---|---|---|---|
| Metformin granulate | Ex. 1a | Ex. 2a | Ex. 3a | Ex. 4a | Ex. 5a |
| Vildagliptin | 50 | 50 | 50 | 50 | 50 |
| Microcrystalline cellulose | - | - | 46 | - | - |
| Lactose monohydrate | - | - | - | 10 | - |
| Mannitol | - | - | - | - | 10 |
| Crospovidone | - | - | - | - | - |
| Mg-stearate | 11 | 11 | 11 | 11 | 11 |

Metformin granulate of Example 3a is not a metformin granulate comprising both a binder and a diluent. The compression mixture of example 3 is not falling within the subject-matter for which protection is sought.

**Table 2 (continued):**

| **Compression mixture** | | | | | |
|---|---|---|---|---|---|
| Example | 6 | 7 | 8 | 9 | 10 |

| **Components (mg)** | | | | | |
|---|---|---|---|---|---|
| Metformin granulate | Ex. 6a | Ex. 7a | Ex. 8a | Ex. 9a | Ex. 10a |
| Vildagliptin | 50 | 50 | 50 | 50 | 50 |
| Microcrystalline cellulose | 20 | 50 | - | - | 34 |
| Lactose monohydrate | - | - | - | 60 | - |
| Mannitol | - | - | 50 | - | - |
| Crospovidone | - | - | - | 10 | 10 |
| Mg-stearate | 11 | 11 | 11 | 11 | 11 |

### Compression of tablets

The compression mixtures (1-10) were compressed into oval tablets with a theoretical weight of 1160 mg (1-6), 1260 mg (7-9) or 1170 mg (10).

### Preparation of metformin granulate using high shear granulator

Hereinabove mentioned examples (Examples 1-10) were also carried out using high shear granulator instead of fluid bed granulator. Physical properties and quality of compressed tablets have met the requirements of the quality control test (weight variation, friability, hardness, disintegration, dissolution profile in selected media). However, fluid bed granulation provides tablets with preferable characteristics.

### EXAMPLES 11-15

Further examples present manufacturing process of vildagliptin granules by wet granulation.

**Table 3 - examples of vildagliptin granulate compositions:**

| **Granulate** | | | | | |
|---|---|---|---|---|---|
| Example | 11 | 12 | 13 | 14 | 15 |

| **Components (mg)** | | | | | |
|---|---|---|---|---|---|
| Vildagliptin | 50 | 50 | 50 | 50 | 50 |
| Microcrystalline cellulose | 35 | - | - | 45 | 20 |
| Mannitol | - | 35 | - | - | 25 |
| Lactose monohydrate | - | - | 35 | - | - |
| Low substituted HPC | 10 | 10 | 10 | - | - |
| HPC | 5 | 5 | 5 | 5 | 5 |
| Ethanol 96% | q.s. | q.s. | q.s. | q.s. | q.s. |

Solid components shown in Table 3 were homogenized in a high shear mixer or in a fluid bed granulator, and ethanol 96% was sprayed over this mixture. Alternatively, a part or a whole amount of binder can be dissolved in the granulation liquid which is sprayed onto the powder mixture shown in Table 3. The obtained granules were dried in a drying chamber or fluid bed dryer.

### EXAMPLES 16-20

Further examples present manufacturing process of vildagliptin granules by dry granulation.

**Table 4 - examples of vildagliptin granulate compositions:**

| **Granulate** | | | | | |
|---|---|---|---|---|---|
| Example | 16 | 17 | 18 | 19 | 20 |

| **Components (mg)** | | | | | |
|---|---|---|---|---|---|
| Vildagliptin | 50 | 50 | 50 | 50 | 50 |
| Microcrystalline cellulose | 50 | - | - | 30 | 30 |
| Mannitol | - | 50 | - | 20 | 10 |
| Lactose monohydrate | - | - | 50 | - | - |
| Low substituted HPC | - | - | - | - | 10 |

Solid components shown in Table 4 were homogenised in a container mixer. Homogenised material was compacted using a roller compactor. The compacted material was sieved to prepare sieved granulate.

### EXAMPLES 21-25

Further examples present manufacturing process of vildagliptin granules by hot melt granulation.

**Table 5 - examples of vildagliptin granulate compositions:**

| **Granulate** | | | | | |
|---|---|---|---|---|---|
| Example | 21 | 22 | 23 | 24 | 25 |

| **Components (mg)** | | | | | |
|---|---|---|---|---|---|
| Vildagliptin | 50 | 50 | 50 | 50 | 50 |
| Low substituted HPC | 10 | 10 | 10 | 15 | 7,5 |
| PEG 6000 | 10 | 10 | 15 | 9 | 12,5 |
| Lactose monohydrate | 30 | - | 25 | - | 15 |
| Mannitol | - | 30 | - | 26 | 15 |

All solid components shown in Table 5 were mixed in the high shear or fluid bed mixer and heated to melting point of the binder while mixing. The mixing continued until granulate was obtained. The obtained granules were sieved by using oscillating sieve.

Alternatively, melted binder (PEG 6000) can be sprayed onto the powder mixture while mixing.

### EXAMPLES 26-35

Further examples present compression mixture compositions containing both metformin and vildagliptin in the form of granules.

**Table 6 - examples of compression mixture compositions containing both active ingredients in granulate form:**

| **Compression mixture** | | | | | |
|---|---|---|---|---|---|
| Example | 26 | 27 | 28 | 29 | 30 |

| **Components (mg)*** | | | | | |
|---|---|---|---|---|---|
| Metformin granulate | Ex. 1a | Ex. 2a | Ex. 3a | Ex. 4a | Ex. 5a |
| Vildagliptin granulate | Ex. 11 | Ex. 12 | Ex. 14 | Ex. 15 | Ex. 16 |
| Microcrystalline cellulose | - | - | 46 | - | - |
| Lactose monohydrate | - | - | - | 10 | - |
| Mannitol | - | - | - | - | 10 |
| Crospovidone | - | - | - | - | - |
| Mg-stearate | 11 | 11 | 11 | 11 | 11 |

| | | | | | |
|---|---|---|---|---|---|
| ^{∗}... the composition and quantity of metformin and vildagliptin granulate is given in corresponding examples above (Table 1 and Table 3) | | | | | |

Metformin granulate of Example 3a is not a metformin granulate comprising both a binder and a diluent. The compression mixture of example 28 is not falling within the subject-matter for which protection is sought.

**Table 6 (continued):**

| **Compression mixture** | | | | | |
|---|---|---|---|---|---|
| Example | 31 | 32 | 33 | 341 | 35 |

| **Components (mg)*** | | | | | |
|---|---|---|---|---|---|
| Metformin granulate | Ex. 6a | Ex. 7a | Ex. 8a | Ex. 9a | Ex. 10a |
| Vildagliptin granulate (Example) | Ex. 17 | Ex. 19 | Ex. 21 | Ex. 23 | Ex. 24 |
| Microcrystalline cellulose | 20 | - | - | 10 | 34 |
| Lactose monohydrate | - | - | - | - | - |
| Mannitol | - | - | - | - | - |
| Crospovidone | - | - | - | 10 | 10 |
| Mg-stearate | 11 | 11 | 11 | 11 | 11 |

| | | | | | |
|---|---|---|---|---|---|
| *... the composition and quantity of metformin and vildagliptin granulate is given in corresponding examples above (Table 2 and Table 4) | | | | | |

### Preparation of compression mixtures

The components shown in Table 6 were mixed in the container mixer to obtain final compression mixture (Examples 26-35).

### Compression of tablets

The compression mixtures (26-35) were compressed into oval tablets with a theoretical weight of 1210 mg (26-31), 1260 mg (32-34) or 1220 mg (35).

### Preparation of metformin granulate using high shear granulator

Hereinabove mentioned examples (Examples 26-35) were also carried out in such a way that metformin granulate was prepared in a high shear granulator. Use of high shear technology for metformin granulation resulted in low hardness of produced tablets.

### PVP EXAMPLES 1-6

Further examples evaluate feasibility of the process using polyvinylpyrrolidone (PVP) as binder.

### Preparation of metformin granulate

**Table 7 - examples of metformin granulate compositions 1b-6b:**

| **Granulate** | | | | | | |
|---|---|---|---|---|---|---|
| Metformin granulate | Ex. 1b | Ex. 2b | Ex. 3b | Ex. 4b | Ex. 5b | Ex. 6b |

| **Components (mg)** | | | | | | |
|---|---|---|---|---|---|---|
| Metformin hydrochloride | 1000 | 1000 | 1000 | 1000 | 1000 | 1000 |
| Microcrystalline cellulose | 20 | 20 | - | - | 44 | 44 |
| Mannitol | 24 | 24 | - | - | - | - |
| PVP | 55 | 55 | 53 | 53 | 55 | 55 |
| Purified water | q.s | q.s. | q.s. | q.s. | q.s. | q.s. |

The solid components shown in Table 7 (without binder) were homogenized in a fluid bed granulator or high shear granulator. Afterwards, binder (PVP) dissolved in the granulation liquid was sprayed onto the powder mixture. Alternatively, a part or whole amount of binder can be put into the powder mixture and purified water is sprayed over this mixture. The obtained granulate was dried in fluid bed dryer at a specified temperature.

### Preparation of compression mixtures

The components shown in Table 8 were mixed in the container mixer to obtain final compression mixture (PVP examples 1-6).

**Table 8 - components added to the metformin granulates:**

| **Compression mixture** | | | | | | |
|---|---|---|---|---|---|---|
| PVP example | 1 | 2 | 3 | 4 | 5 | 6 |

| **Components (mg)** | | | | | | |
|---|---|---|---|---|---|---|
| Metformin granulate | Ex. 1b | Ex. 2b | Ex. 3b | Ex. 4b | Ex. 5b | Ex. 6b |
| Vildagliptin | 50 | 50 - | 50 | 50 - | 50 | - |
| Vildagliptin granulate (Ex. 12) | - | 100 | - | 100 | | 100 |
| Microcrystalline cellulose | - | - | 46 | 46 | - | - |
| Mg-stearat | 11 | 11 | 11 | 11 | 11 | 11 |

PVP examples 1 to 6 are not falling within the subject-matter for which protection is sought.

Vildagliptin granulate used in the above examples was produced as described in Example 12 - vildagliptin granules production by wet granulation.

### Compression of tablets

The compression mixtures (1-6) were compressed into oval tablets with a theoretical weight of 1160 mg (1, 3, 5) or 1210 mg (2, 4, 6).

Production of tablets was achieved. Compression mixtures prepared using PVP as a binder had slightly lower compressibility and compactibility. Therefore, production of tablets with optimal hardness was not possible.

### EXAMPLES 36-38

Further examples present possible compositions of film coating suspension.

**Table 9 - examples of film coating suspension compositions:**

| **Film coating suspension** | | | |
|---|---|---|---|
| Example | 36 | 37 | 38 |

| **Composition (mg)** | | | |
|---|---|---|---|
| OPADRY^{®} premix | 98.2 | | |
| OPADRY^{®} II premix | | 98.2 | |
| Kollicoat^{®} protect | | | 60.2 |
| Talc | | | 16 |
| Titanium Dioxide | | | 22 |
| Colouring agent (iron oxide) | 1.8 | 1.8 | 1.8 |
| Purified water | q.s. | q.s. | q.s. |

Film coating was optionally applied in an amount of approximately 2.5% by weight of a tablet core.

### EXAMPLES 39 AND 40

**Table 10 - composition of Example 39:**

| **Components (mg)** | | | |
|---|---|---|---|
| **Compression mixture** | **Metformin granulate** | Metformin hydrochloride | 1000 |
| | | Microcrystalline cellulose* | 44 |
| | | Hydroxypropyl cellulose | 55 |
| | | Purified water | q.s. |
| | Vildagliptin | | 50 |
| | Magnesium stearate | | 11 |
| **Film coating** | | Opadry premix | 26.3 |
| | | Iron oxide - Yellow | 1.7 |
| | | Purified water | q.s. |

| | | | |
|---|---|---|---|
| ^{∗}Microcrystalline cellulose commercially available as Ceolus^{™} grade KG-802 was used. | | | |

Metformin granulate and compression mixture were produced as described in Examples 1-10 section. The compression mixture was compressed into oval tablets with theoretical weight of 1160 mg. Compression force from 30 to 45 kN was applied during the process and tablet cores with hardness between 130 and 190 N were obtained. Tablet cores were further film coated and film coated tablets with weight of approximately 1188 mg were obtained.

**Table 11 - composition of Example 40:**

| | | | |
|---|---|---|---|
| **Compression mixture** | **Metformin granulate** | Metformin hydrochloride | 1000 |
| | | Microcrystalline cellulose* | 44 |
| | | Hydroxypropyl cellulose | 55 |
| | | Purified water | q.s. |
| | **Vildagliptin granulate** | Vildagliptin | 50 |
| | | Mannitol | 35 |
| | | Low substituted HPC | 10 |
| | | Hydroxypropyl cellulose | 5 |
| | | Ethanol | q.s. |
| | Magnesium stearate | | 11 |
| **Film coating** | | Opadry^{®} premix | 28,20 |
| | | Iron oxide - Yellow | 1,80 |
| | | Purified water | q.s. |

| | | | |
|---|---|---|---|
| ^{∗}Microcrystalline cellulose commercially available as Ceolus^{™} grade KG-802 was used. | | | |

Metformin granulate was produced as described in Examples 1-10 section.

Vildagliptin granulate was produced by wet granulation process in fluid bed granulator as described in Examples 11-15 section.

Metformin granulate and vildagliptin granulate were mixed together with magnesium stearate in the container mixer to obtain final compression mixture. The compression mixture was afterwards compressed into oval tablets with a theoretical weight of 1210 mg. Compression force from 18 to 30 kN was applied during the process and tablet cores with hardness between 180 and 250 N were obtained. Tablet cores were further film coated and film coated tablets with weight of approximately 1240 mg were obtained.

### EXAMPLE 41

Comparison of stress stability of Example 39 and Example 40 formulations with reference product (Eucreas^{®} 50mg/1000mg film coated tablets) was done. Tablets were closed in glass vials and stored at 40 °C and 75 % relative humidity for 3 months.

In all of the above mentioned formulations after 3 month stress stability testing metformin related substances were below the reporting threshold.

**Table 12 - vildagliptin related substances increase after 3 month stress stability exposure at 40 °C and 75 % relative humidity:**

| | **Increase of vildagliptin related substances (%)** |
|---|---|
| Example 39 | 1.24 |
| Example 40 | 1.23 |
| Eucreas^{®} (Batch WO008) | 1.38 |

### COMPARATIVE EXAMPLE 1

Further example evaluates dry granulation as metformin granules production possibility.

**Table 13 - metformin granulate composition:**

| **Components (mg)** | |
|---|---|
| Metformin hydrochloride | 1000 |
| Hydroxypropyl cellulose | 99 |

Solid components shown in Table 13 were homogenised in a container mixer. Homogenised material was compacted using a roller compactor. The compacted material was sieved to prepare sieved granulate.

**Table 14 - final compression mixture composition:**

| **Components (mg)** | |
|---|---|
| Metformin granulate | 1099 |
| Vildagliptin granulate - Example 12 | 100 |
| Magnesium stearate | 11 |

Vildagliptin granulate used in this example was produced as described in Example 12 - vildagliptin granules production by wet granulation.

The components shown in Table 14 were mixed in the container mixer to obtain final compression mixture which was further compressed into oval tablets with theoretical weight of 1210 mg.

The compression mixture obtained in such a manner showed poor compressibility, compactibility and flow properties. Therefore, tableting process was very difficult and production of tablet cores with proper hardness and friability properties was practically impossible. Capping phenomenon was also noted.

### COMPARATIVE EXAMPLE 2

Further example evaluates melt granulation as metformin granules production possibility.

**Table 15 - metformin granulate composition:**

| **Components (mg)** | |
|---|---|
| Metformin hydrochloride | 1000 |
| PEG 4000 powder | 42.9 |

Solid components shown in Table 15 were mixed in the fluid bed mixer and heated to the melting point of the binder while mixing. The mixing continued until granulate was obtained. The obtained granules were sieved by using oscillating sieve.

**Table 16 - final compression mixture composition:**

| **Components (mg)** | |
|---|---|
| Metformin granulate | 1042.9 |
| Avicel PH112 | 56.1 |
| Vildagliptin granulate | 100 |
| Magnesium stearate | 11 |

Vildagliptin granulate used in this example was produced as described in Example 12 - vildagliptin granules production by wet granulation.

The components shown in Table 16 were mixed in the container mixer to obtain final compression mixture which was further compressed.

The compression mixture obtained in such a manner showed very poor compressibility and compatibility which is why production of persistent and compact tablets was impossible.

### COMPARATIVE EXAMPLE 3

Further example evaluates production possibility without metformin granulation.

**Table 17 - compression mixture composition:**

| **Components (mg)** | |
|---|---|
| Metformin hydrochloride | 1000 |
| Hydroxypropyl cellulose | 87 |
| Colloidal silicon dioxide | 12 |
| Vildagliptin granulate - Example 12 | 100 |
| Magnesium stearate | 11 |

Vildagliptin granulate used in this example was produced as described in Example 12 - vildagliptin granules production by wet granulation.

The components shown in Table 17 were mixed in the container mixer to obtain final compression mixture.

Compression process was in this case impossible due to poor flow properties of the mixture.

### EXAMPLES 42-47

**Table 18 - composition of Example 42:**

| **Components (mg)** | | | |
|---|---|---|---|
| **Compression mixture** | **Metformin granulate** | Metformin hydrochloride | 1000 |
| | | Microcrystalline cellulose | 114 |
| | | Hydroxypropyl cellulose | 60 |
| | | Purified water | q.s. |
| | Vildagliptin | | 50 |
| | Magnesium stearate | | 11 |
| **Film coating** | | Opadry premix | 28.2 |
| | | Iron oxide - Yellow | 1.8 |
| | | Purified water | q.s. |

Metformin granulate and compression mixture were produced as described in Examples 1-10 section. The compression mixture was compressed into oval tablets with theoretical weight of 1235 mg. Tablet cores were further film coated and film coated tablets with weight of approximately 1265 mg were obtained.

**Table 19 - composition of Example 43:**

| | | | |
|---|---|---|---|
| **Compression mixture** | **Metformin granulate** | Metformin hydrochloride | 1000 |
| | | Microcrystalline cellulose | 68 |
| | | Hydroxypropyl cellulose | 56 |
| | | Purified water | q.s. |
| | **Vildagliptin granulate** | Vildagliptin | 50 |
| | | Mannitol | 35 |
| | | Low substituted HPC | 10 |
| | | Hydroxypropyl cellulose | 5 |
| | | Ethanol | q.s. |
| | Magnesium stearate | | 11 |
| **Film coating** | | Opadry^{®} premix | 28,20 |
| | | Iron oxide - Yellow | 1,80 |
| | | Purified water | q.s. |

**Table 20 - composition of Example 44:**

| | | | |
|---|---|---|---|
| **Compression mixture** | **Metformin granulate** | Metformin hydrochloride | 1000 |
| | | Microcrystalline cellulose | 59 |
| | | Hydroxypropyl cellulose | 60 |
| | | Purified water | q.s. |
| | **Vildagliptin granulate** | Vildagliptin | 50 |
| | | Mannitol | 45 |
| | | Hydroxypropyl cellulose | 5 |
| | | Ethanol | q.s. |
| | Magnesium stearate | | 11 |
| **Film coating** | | Opadry^{®} premix | 28,20 |
| | | Iron oxide - Yellow | 1,80 |
| | | Purified water | q.s. |

**Table 21 - composition of Example 45:**

| | | | |
|---|---|---|---|
| **Compression mixture** | **Metformin granulate** | Metformin hydrochloride | 1000 |
| | | Microcrystalline cellulose | 59 |
| | | Hydroxypropyl cellulose | 60 |
| | | Purified water | q.s. |
| | **Vildagliptin granulate** | Vildagliptin | 50 |
| | | Mannitol | 35 |
| | | Low substituted HPC | 10 |
| | | Hydroxypropyl cellulose | 5 |
| | | Ethanol | q.s. |
| | Magnesium stearate | | 11 |
| **Film coating** | | Opadry^{®} premix | 28,20 |
| | | Iron oxide - Yellow | 1,80 |
| | | Purified water | q.s. |

**Table 22 - composition of Example 46:**

| | | | |
|---|---|---|---|
| **Compression mixture** | **Metformin granulate** | Metformin hydrochloride | 1000 |
| | | Mannitol* | 59 |
| | | Hydroxypropyl cellulose | 60 |
| | | Purified water | q.s. |
| | **Vildagliptin granulate** | Vildagliptin | 50 |
| | | Mannitol* | 35 |
| | | Low substituted HPC | 10 |
| | | Hydroxypropyl cellulose | 5 |
| | | Ethanol | q.s. |
| | Magnesium stearate | | 11 |
| **Film coating** | | Opadry^{®} premix | 28,20 |
| | | Iron oxide - Yellow | 1,80 |
| | | Purified water | q.s. |

| | | | |
|---|---|---|---|
| *Mannitol commercially available as Parteck^{®} grade M200 was used. | | | |

**Table 23 - composition of Example 47:**

| | | | |
|---|---|---|---|
| **Compression mixture** | **Metformin granulate** | Metformin hydrochloride | 1000 |
| | | Mannitol* | 59 |
| | | Hydroxypropyl cellulose | 60 |
| | | Purified water | q.s. |
| | **Vildagliptin granulate** | Vildagliptin | 50 |
| | | Mannitol* | 45 |
| | | Hydroxypropyl cellulose | 5 |
| | | Ethanol | q.s. |
| | Magnesium stearate | | 11 |
| **Film coating** | | Opadry^{®} premix | 28,20 |
| | | Iron oxide - Yellow | 1,80 |
| | | Purified water | q.s. |

| | | | |
|---|---|---|---|
| *Mannitol commercially available as Parteck^{®} grade M200 was used. | | | |

Metformin granulate was produced as described in Examples 1-10 section.

Vildagliptin granulate was produced by wet granulation process in fluid bed granulator as described in Examples 11-15 section.

Metformin granulate and vildagliptin granulate were mixed together with magnesium stearate in the container mixer to obtain final compression mixture. The compression mixture was afterwards compressed into oval tablets with a theoretical weight of 1230 mg (Ex. 44-47) or 1235 mg (Ex. 43). Tablet cores were further film coated and film coated tablets with weight of approximately 1260 mg (Ex. 44-47) or 1265 mg (Ex. 43) mg were obtained.

## Claims

1. Solid pharmaceutical composition comprising metformin in the form of granules and vildagliptin in the form of drug particles or in the form of granules and at least one pharmaceutically acceptable excipient,
said solid pharmaceutical composition including diluent, said diluent being selected from mannitol, lactose, sorbitol and microcrystalline cellulose and combinations thereof, and
said solid pharmaceutical composition including binder, said binder being selected from hydroxypropyl cellulose (HPC), hydroxypropyl methylcellulose (HPMC), and hydroxyethyl cellulose (HEC) and combinations thereof,
wherein metformin in the form of granules is prepared by wet granulation, and
wherein metformin in the form of granules comprises both, a binder and a diluent.

2. Solid pharmaceutical composition according to claim 1, wherein pharmaceutically acceptable excipient is selected from one or more binders, one or more diluents and/or one or more lubricants.

3. Solid pharmaceutical composition according to claim 2, wherein the pharmaceutically acceptable excipient includes both, a binder and a diluent.

4. Solid pharmaceutical composition according to claim 1, wherein the amount of binder is between 0.5 to 30% by weight, preferably from 1 to 20% by weight of the composition and the amount of diluent is from 0.5 to 25% by weight, preferably from 1 to 15% by weight of the composition.

5. Solid pharmaceutical composition according to any of preceding claims, wherein the weight ratio between diluent and binder is from 2.5:1 to 1:5, preferably from 2:1 to 1:4, more preferably from 1.5:1 to 1:3.

6. Solid pharmaceutical composition according to any of preceding claims, wherein diluent is mannitol and/or microcrystalline cellulose, and/or
wherein binder is hydroxypropyl cellulose, and/or
wherein lubricants are selected from metal salt of stearic acid, stearic acid, sodium stearyl fumarate or their mixture, preferably magnesium stearate and the amount is from about 0.1% to 5% by weight of the composition, preferably from 0.25% to 3% by weight of the composition.

7. Solid pharmaceutical composition according to any of the preceding claims, wherein the amount of vildagliptin or acceptable salt thereof is between 1% by weight and 30% by weight, preferably from 1.5% by weight to 15% by weight of the composition and the amount of metformin or acceptable salt thereof is between 60% by weight and 98% by weight, preferably between 70% by weight and 95% by weight, more preferably between 70 % by weight and 80% by weight of the composition.

8. Solid pharmaceutical composition according to any of the preceding claims, wherein the average particle size of metformin or acceptable salt thereof is between 40 to 240 µm, preferably between 60 to 180 µm and the average particle size of vildagliptin or acceptable salt thereof is between 10 and 250 µm, preferably between 20 and 150 µm, average particle size being determined by laser diffraction method.

9. The process for manufacturing of the solid pharmaceutical composition, said solid pharmaceutical composition including diluent, said diluent being selected from mannitol, lactose, sorbitol and microcrystalline cellulose and combinations thereof, and
said solid pharmaceutical composition including binder, said binder being selected from hydroxypropyl cellulose (HPC), hydroxypropyl methylcellulose (HPMC), and hydroxyethyl cellulose (HEC) and combinations thereof,
said process comprising the following steps:
i) production of metformin granulate by wet granulation, wherein metformin granulate of step i) comprises both, a binder and a diluent,
ii) optionally production of vildagliptin granulate,
iii) blending vildagliptin in the form of drug particles or in the form of granulate and optionally other pharmaceutically acceptable excipients with metformin granulate,
iv) optionally blending lubricant with the mixture obtained in step iii) to obtain compression mixture,
v) compressing the resulting compression mixture to form tablets, preferably tablets in unit dosage form,
vi) optionally film coating of tablets obtained in step v).

10. The process for manufacturing of the solid pharmaceutical composition, said solid pharmaceutical composition including diluent, said diluent being selected from mannitol, lactose, sorbitol and microcrystalline cellulose and combinations thereof, and
said solid pharmaceutical composition including binder, said binder being selected from hydroxypropyl cellulose (HPC), hydroxypropyl methylcellulose (HPMC), and hydroxyethyl cellulose (HEC) and combinations thereof,
said process comprising the following steps:
i) production of metformin granulate by wet granulation, wherein metformin granulate of step i) comprises both, a binder and a diluent,
ii) production of vildagliptin granulate,
iii) blending vildagliptin in the form of optionally coated granulate and optionally other pharmaceutically acceptable excipients with metformin granulate,
iv) optionally blending lubricant with the mixture obtained in step iii) to obtain compression mixture,
v) compressing the resulting compression mixture to form tablets, preferably tablets in unit dosage form,
vi) optionally film coating of tablets obtained in step v).

11. The process for manufacturing of the solid pharmaceutical composition according to claims 9 or 10, wherein weight ratio between diluent and binder in the composition is from 2.5:1 to 1:5, preferably from 2:1 to 1:4, more preferably from 1.5:1 to 1:3.

12. The process for manufacturing of the solid pharmaceutical composition according to any one of claims 9 to 11, wherein diluent is mannitol and/or microcrystalline cellulose and binder is hydroxypropyl cellulose.

13. The process for manufacturing of the solid pharmaceutical composition according to claims 9-12, wherein the bulk volume of metformin granulate of step i) is in the range from 1.5 mL/g to 3.2 mL/g, preferably in the range from 1.8 mL/g and 3.0 mL/g, and/or
wherein the loss on drying value (LOD) of metformin granulate of step i) is in the range of 0.025% to 1.5 %, preferably of 0.05% to 0.8%, and/or
wherein at least 40 weight %, preferably at least 60 weight % metformin granules obtained in step i) are in the particle size range from 125 to 500 µm measured by sieve analysis.

14. The process for manufacturing of the solid pharmaceutical composition according to claims 9-13, wherein at least 40 weight %, preferably at least 50 weight % vildagliptin granulate obtained in step ii) are in the particle size range from 71 to 250 µm measured by sieve analysis, and/or
wherein the bulk volume of vildagliptin granulate of step ii) is in the range from 0.5 mL/g to 3.0 mL/g, preferably in the range from 1.0 mL/g to 2.5 mL/g, and/or
wherein the loss on drying value (LOD) of vildagliptin granulate of step ii) is in the range of 0.05% to 1.8%, preferably from 0.1% to 0.9%.

15. The process for manufacturing of the solid pharmaceutical composition according to claims 9-14, wherein:
- the loss on drying value (LOD) of compression mixture of step iv) is in the range of 0.05% to 1.7%, preferably of 0.1% to 0.8%,
- bulk volume of the compression mixture of step iv) is in a range of 1.5 mL/g to 3.2 mL/g, preferably in a range of 1.8 mL/g to 3.0 mL/g, most preferably about 2.5 mL/g,
- particle size of the compression mixture of step iv), wherein at least 40 weight %, preferably at least 50 weight % of compression mixture obtained in step iv) are in the range from 125 to 500 µm measured by sieve analysis.

## Patentansprüche

1. Feste pharmazeutische Zusammensetzung umfassend Metformin in der Form von Granula und Vildagliptin in der Form von Arzneistoffteilchen oder in der Form von Granula und mindestens einen pharmazeutisch verträglichen Exzipienten,
wobei die feste pharmazeutische Zusammensetzung Verdünnungsmittel einschließt, wobei das Verdünnungsmittel aus Mannitol, Lactose, Sorbitol und mikrokristalliner Cellulose und Kombinationen davon ausgewählt ist, und
wobei die feste pharmazeutische Zusammensetzung Bindemittel einschließt, wobei das Bindemittel aus Hydroxypropylcellulose (HPC), Hydroxypropylmethylcellulose (HPMC) und Hydroxyethylcellulose (HEC) und Kombinationen davon ausgewählt ist, wobei Metformin in der Form von Granula durch Feuchtgranulierung hergestellt wird, und
wobei Metformin in der Form von Granula sowohl ein Bindemittel als auch ein Verdünnungsmittel umfasst.

2. Feste pharmazeutische Zusammensetzung gemäß Anspruch 1, wobei pharmazeutisch verträglicher Exzipient aus einem oder mehr Bindemitteln, einem oder mehr Verdünnungsmitteln und/oder einem oder mehr Gleitmitteln ausgewählt ist.

3. Feste pharmazeutische Zusammensetzung gemäß Anspruch 2, wobei der pharmazeutisch verträgliche Exzipient sowohl ein Bindemittel als auch ein Verdünnungsmittel einschließt.

4. Feste pharmazeutische Zusammensetzung gemäß Anspruch 1, wobei die Menge an Bindemittel zwischen 0,5 bis 30 Gew.-% liegt, bevorzugt 1 bis 20 Gew.-% der Zusammensetzung beträgt und die Menge an Verdünnungsmittel 0,5 bis 25 Gew.-%, bevorzugt 1 bis 15 Gew.-% der Zusammensetzung beträgt.

5. Feste pharmazeutische Zusammensetzung gemäß einem der vorhergehenden Ansprüche, wobei das Gewichtsverhältnis zwischen Verdünnungsmittel und Bindemittel 2,5:1 bis 1:5, bevorzugt 2:1 bis 1:4, stärker bevorzugt 1,5:1 bis 1:3 beträgt.

6. Feste pharmazeutische Zusammensetzung gemäß einem der vorhergehenden Ansprüche, wobei Verdünnungsmittel Mannitol und/oder mikrokristalline Cellulose ist, und/oder
wobei Bindemittel Hydroxypropylcellulose ist, und/oder
wobei Gleitmittel aus Metallsalz von Stearinsure, Stearinsäure, Natriumstearylfumarat oder deren Gemisch, bevorzugt Magnesiumstearat ausgewählt sind, und die Menge etwa 0,1 Gew.-% bis 5 Gew.-% der Zusammensetzung, bevorzugt 0,25 Gew.-% bis 3 Gew.-% der Zusammensetzung beträgt.

7. Feste pharmazeutische Zusammensetzung gemäß einem der vorhergehenden Ansprüche, wobei die Menge an Vildagliptin oder verträglichem Salz davon zwischen 1 Gew.-% und 30 Gew.-% liegt, bevorzugt 1,5 Gew.-% bis 15 Gew.-% der Zusammensetzung beträgt und die Menge an Metformin oder verträglichem Salz davon zwischen 60 Gew.-% und 98 Gew.-%, bevorzugt zwischen 70 Gew.-% und 95 Gew.-%, stärker bevorzugt zwischen 70 Gew.-% und 80 Gew.-% der Zusammensetzung liegt.

8. Feste pharmazeutische Zusammensetzung gemäß einem der vorhergehenden Ansprüche, wobei die mittlere Teilchengröße von Metformin oder verträglichem Salz davon zwischen 40 bis 240 µm, bevorzugt zwischen 60 bis 180 µm liegt und die mittlere Teilchengröße von Vildagliptin oder verträglichem Salz davon zwischen 10 und 150 µm, bevorzugt zwischen 20 und 150 µm liegt, wobei mittlere Teilchengröße durch ein Laserbeugungsverfahren bestimmt wird.

9. Verfahren zum Herstellen der festen pharmazeutischen Zusammensetzung, wobei die feste pharmazeutische Zusammensetzung Verdünnungsmittel einschließt, wobei das Verdünnungsmittel aus Mannitol, Lactose, Sorbitol und mikrokristalliner Cellulose und Kombinationen davon ausgewählt ist, und
wobei die feste pharmazeutische Zusammensetzung Bindemittel einschließt, wobei das Bindemittel aus Hydroxypropylcellulose (HPC), Hydroxypropylmethylcellulose (HPMC) und Hydroxyethylcellulose (HEC) und Kombinationen davon ausgewählt ist, wobei das Verfahren die folgenden Schritte umfasst:
i) Herstellung von Metformin-Granulat durch Feuchtgranulierung, wobei Metformin-Granulat von Schritt i) sowohl ein Bindemittel als auch ein Verdünnungsmittel umfasst,
ii) gegebenenfalls Herstellung von Vildagliptin-Granulat,
iii) Mischen von Vildagliptin in der Form von Arzneistoffteilchen oder in der Form von Granulat und gegebenenfalls anderen pharmazeutisch verträglichen Exzipienten mit Metformin-Granulat,
iv) gegebenenfalls Mischen von Gleitmittel mit dem in Schritt iii) erhaltenen Gemisch, wobei Verpressungsgemisch erhalten wird,
v) Verpressen des resultierenden Verpressungsgemisches, wobei Tabletten, bevorzugt Tabletten in Dosierungseinheitform gebildet werden,
vi) gegebenenfalls Überziehen von in Schritt v) erhaltenen Tabletten mit einem Film.

10. Verfahren zum Herstellen der festen pharmazeutischen Zusammensetzung, wobei die feste pharmazeutische Zusammensetzung Verdünnungsmittel einschließt, wobei das Verdünnungsmittel aus Mannitol, Lactose, Sorbitol und mikrokristalliner Cellulose und Kombinationen davon ausgewählt ist, und
wobei die feste pharmazeutische Zusammensetzung Bindemittel einschließt, wobei das Bindemittel aus Hydroxypropylcellulose (HPC), Hydroxypropylmethylcellulose (HPMC) und Hydroxyethylcellulose (HEC) und Kombinationen davon ausgewählt ist, wobei das Verfahren die folgenden Schritte umfasst:
i) Herstellung von Metformin-Granulat durch Feuchtgranulierung, wobei Metformin-Granulat von Schritt i) sowohl ein Bindemittel als auch ein Verdünnungsmittel umfasst,
ii) Herstellung von Vildagliptin-Granulat,
iii) Mischen von Vildagliptin in der Form von gegebenenfalls überzogenem Granulat und gegebenenfalls anderen pharmazeutisch verträglichen Exzipienten mit Metformin-Granulat,
iv) gegebenenfalls Mischen von Gleitmittel mit dem in Schritt iii) erhaltenen Gemisch, wobei Verpressungsgemisch erhalten wird,
v) Verpressen des resultierenden Verpressungsgemisches, wobei Tabletten, bevorzugt Tabletten in Dosierungseinheitform gebildet werden,
vi) gegebenenfalls Überziehen von in Schritt v) erhaltenen Tabletten mit einem Film.

11. Verfahren zum Herstellen der festen pharmazeutischen Zusammensetzung gemäß den Ansprüchen 9 oder 10, wobei ein Gewichtsverhältnis zwischen Verdünnungsmittel und Bindemittel in der Zusammensetzung 2,5:1 bis 1:5, bevorzugt 2:1 bis 1:4, stärker bevorzugt 1,5:1 bis 1:3 beträgt.

12. Verfahren zum Herstellen der festen pharmazeutischen Zusammensetzung gemäß einem der Ansprüche 9 bis 11, wobei Verdünnungsmittel Mannitol und/oder mikrokristalline Cellulose ist und Bindemittel Hydroxypropylcellulose ist.

13. Verfahren zum Herstellen der festen pharmazeutischen Zusammensetzung gemäß den Ansprüchen 9-12, wobei das Schüttvolumen von Metformin-Granulat von Schritt i) im Bereich von 1,5 mL/g bis 3,2 mL/g, bevorzugt im Bereich von 1,8 mL/g und 3,0 mL/g liegt, und/oder
wobei der Wert des Trocknungsverlusts (LOD) von Metformin-Granulat von Schritt i) im Bereich von 0,025 % bis 1,5 %, bevorzugt von 0,05 % bis 0,8 % liegt, und/oder
wobei mindestens 40 Gew.-%, bevorzugt mindestens 60 Gew.-% von in Schritt i) erhaltenen Metformin-Granula im Teilchengrößenbereich von 125 bis 500 µm, gemessen durch Siebanalyse, liegen.

14. Verfahren zum Herstellen der festen pharmazeutischen Zusammensetzung gemäß den Ansprüchen 9-13, wobei mindestens 40 Gew.-%, bevorzugt mindestens 50 Gew.-% von in Schritt ii) erhaltenem Vildagliptin-Granulat im Teilchengrößenbereich von 71 bis 250 µm, gemessen durch Siebanalyse, liegen, und/oder
wobei das Schüttvolumen von Vildagliptin-Granulat von Schritt ii) im Bereich von 0,5 mL/g bis 3,0 mL/g, bevorzugt im Bereich von 1,0 mL/g bis 2,5 mL/g liegt, und/oder wobei der Wert des Trocknungsverlusts (LOD) von Vildagliptin-Granulat von Schritt ii) im Bereich von 0,05 % bis 1,8 %, bevorzugt von 0,1 % bis 0,9 % liegt.

15. Verfahren zum Herstellen der festen pharmazeutischen Zusammensetzung gemäß den Ansprüchen 9-14, wobei:
- der Wert des Trocknungsverlusts (LOD) von Verpressungsgemisch von Schritt iv) im Bereich von 0,05 % bis 1,7 %, bevorzugt von 0,1 % bis 0,8 % liegt,
- ein Schüttvolumen des Verpressungsgemisches von Schritt iv) in einem Bereich von 1,5 mL/g bis 3,2 mL/g, bevorzugt in einem Bereich von 1,8 mL/g bis 3,0 mL/g liegt, am stärksten bevorzugt etwa 2,5 mL/g beträgt,
- Teilchengröße des Verpressungsgemisches von Schritt iv), wobei mindestens 40 Gew.-%, bevorzugt mindestens 50 Gew.-% von in Schritt iv) erhaltenem Verpressungsgemisch im Bereich von 125 bis 500 µm, gemessen durch Siebanalyse, liegen.

## Revendications

1. Composition pharmaceutique solide comprenant de la metformine sous forme de granules et de la vildagliptine sous forme de particules de médicament ou sous forme de granules et au moins un excipient pharmaceutiquement acceptable,
ladite composition pharmaceutique solide comprenant un diluant, ledit diluant étant choisi parmi le mannitol, le lactose, le sorbitol et la cellulose microcristalline et des combinaisons de ceux-ci, et
ladite composition pharmaceutique solide comprenant un liant, ledit liant étant choisi parmi l'hydroxypropylcellulose (HPC), l'hydroxypropylméthylcellulose (HPMC) et l'hydroxyéthylcellulose (HEC) et des combinaisons de celles-ci,
dans laquelle la metformine sous forme de granules est préparée par granulation par voie humide, et
dans laquelle la metformine sous forme de granules comprend à la fois un liant et un diluant.

2. Composition pharmaceutique solide selon la revendication 1, dans laquelle l'excipient pharmaceutiquement acceptable est choisi parmi un ou plusieurs liants, un ou plusieurs diluants et/ou un ou plusieurs lubrifiants.

3. Composition pharmaceutique solide selon la revendication 2, dans laquelle l'excipient pharmaceutiquement acceptable comprend à la fois un liant et un diluant.

4. Composition pharmaceutique solide selon la revendication 1, dans laquelle la quantité de liant est comprise entre 0,5 à 30% en poids, de préférence entre 1 et 20% en poids de la composition et la quantité de diluant est comprise entre 0,5 et 25% en poids, de préférence entre 1 à 15% en poids de la composition.

5. Composition pharmaceutique solide selon l'une quelconque des revendications précédentes, dans laquelle le rapport pondéral entre le diluant et le liant est compris entre 2,5 : 1 et 1 : 5, de préférence entre 2 : 1 et 1 : 4, plus préférablement entre 1,5 : 1 et 1 : 3.

6. Composition pharmaceutique solide selon l'une quelconque des revendications précédentes, dans laquelle le diluant est le mannitol et/ou la cellulose microcristalline, et/ou
dans laquelle le liant est l'hydroxypropylcellulose, et/ou
dans laquelle les lubrifiants sont choisis parmi un sel métallique d'acide stéarique, l'acide stéarique, le stéarylfumarate de sodium ou un mélange de ceux-ci, de préférence le stéarate de magnésium et la quantité est comprise entre environ 0,1% et 5% en poids de la composition, de préférence entre 0,25% et 3% en poids de la composition.

7. Composition pharmaceutique solide selon l'une quelconque des revendications précédentes, dans laquelle la quantité de vildagliptine ou d'un sel acceptable de celle-ci est comprise entre 1% en poids et 30% en poids, de préférence entre 1,5% en poids et 15% en poids de la composition et la quantité de la metformine ou d'un sel acceptable de celle-ci est comprise entre 60% en poids et 98% en poids, de préférence entre 70% en poids et 95% en poids, plus préférablement entre 70% en poids et 80% en poids de la composition.

8. Composition pharmaceutique solide selon l'une quelconque des revendications précédentes, dans laquelle la taille de particule moyenne de la metformine ou d'un sel acceptable de celle-ci est comprise entre 40 et 240 µm, de préférence entre 60 et 180 µm et la taille de particule moyenne de la vildagliptine ou d'un sel acceptable de celle-ci est comprise entre 10 et 250 µm, de préférence entre 20 et 150 µm, la taille de particule moyenne étant déterminée par un procédé de diffraction laser.

9. Procédé de fabrication de la composition pharmaceutique solide, ladite composition pharmaceutique solide comprenant un diluant, ledit diluant étant choisi parmi le mannitol, le lactose, le sorbitol et la cellulose microcristalline et des combinaisons de ceux-ci, et
ladite composition pharmaceutique solide comprenant un liant, ledit liant étant choisi parmi l'hydroxypropylcellulose (HPC), l'hydroxypropylméthylcellulose (HPMC) et l'hydroxyéthylcellulose (HEC) et des combinaisons de celles-ci,
ledit procédé comprenant les étapes suivantes :
i) la production de granulé de metformine par granulation par voie humide, où le granulé de metformine de l'étape i) comprend à la fois un liant et un diluant,
ii) éventuellement la production de granulé de vildagliptine,
iii) le mélange de la vildagliptine sous forme de particules de médicament ou sous forme de granulé et éventuellement d'autres excipients pharmaceutiquement acceptables avec un granulé de metformine,
iv) éventuellement le mélange de lubrifiant avec le mélange obtenu à l'étape iii) pour obtenir un mélange de compression,
v) la compression du mélange de compression résultant pour former des comprimés, de préférence des comprimés sous forme posologique unitaire,
vi) éventuellement le pelliculage de comprimés obtenus à l'étape v).

10. Procédé de fabrication de la composition pharmaceutique solide, ladite composition pharmaceutique solide comprenant un diluant, ledit diluant étant choisi parmi le mannitol, le lactose, le sorbitol et la cellulose microcristalline et des combinaisons de ceux-ci, et
ladite composition pharmaceutique solide comprenant un liant, ledit liant étant choisi parmi l'hydroxypropylcellulose (HPC), l'hydroxypropylméthylcellulose (HPMC) et l'hydroxyéthylcellulose (HEC) et des combinaisons de celles-ci,
ledit procédé comprenant les étapes suivantes :
i) la production de granulé de metformine par granulation par voie humide, où le granulé de metformine de l'étape i) comprend à la fois un liant et un diluant,
ii) la production de granulé de vildagliptine,
iii) le mélange de la vildagliptine sous forme de granulé éventuellement enrobé et éventuellement d'autres excipients pharmaceutiquement acceptables avec le granulé de metformine,
iv) éventuellement le mélange de lubrifiant avec le mélange obtenu à l'étape iii) pour obtenir un mélange de compression,
v) la compression du mélange de compression résultant pour former des comprimés, de préférence des comprimés sous forme posologique unitaire,
vi) éventuellement le pelliculage des comprimés obtenus à l'étape v).

11. Procédé de fabrication de la composition pharmaceutique solide selon la revendication 9 ou 10, dans lequel le rapport pondéral entre le diluant et le liant dans la composition est compris entre 2,5 : 1 et 1 : 5, de préférence entre 2 : 1 et 1 : 4, plus préférablement entre 1,5 : 1 et 1 : 3.

12. Procédé de fabrication de la composition pharmaceutique solide selon l'une quelconque des revendications 9 à 11, dans lequel le diluant est le mannitol et/ou la cellulose microcristalline et le liant est l'hydroxypropylcellulose.

13. Procédé de fabrication de la composition pharmaceutique solide selon les revendications 9 à 12, dans lequel le volume apparent du granulé de metformine de l'étape i) se trouve dans la plage allant de 1,5 mL/g à 3,2 mL/g, de préférence dans la plage allant de 1,8 mL/g à 3,0 mL/g, et/ou
dans lequel la valeur de perte au séchage (LOD) du granulé de metformine de l'étape i) se trouve dans la plage allant de 0,025% à 1,5%, de préférence de 0,05% à 0,8%, et/ou
dans lequel au moins 40% en poids, de préférence au moins 60% en poids de granules de metformine obtenus à l'étape i) se trouvent dans la plage de taille de particule allant de 125 à 500 µm mesurée par analyse granulométrique.

14. Procédé de fabrication de la composition pharmaceutique solide selon les revendications 9 à 13, dans lequel au moins 40% en poids, de préférence au moins 50% en poids du granulé de vildagliptine obtenu à l'étape ii) se trouvent dans la plage de taille de particule allant de 71 à 250 µm mesurée par analyse granulométrique, et/ou
dans lequel le volume apparent de granulé de vildagliptine de l'étape ii) se trouve dans la plage allant de 0,5 mL/g à 3,0 mL/g, de préférence dans la plage allant de 1,0 mL/g à 2,5 mL/g, et/ou
dans lequel la valeur de perte au séchage (LOD) du granulé de vildagliptine de l'étape ii) se trouve dans la plage allant de 0,05% à 1,8%, de préférence de 0,1% à 0,9%.

15. Procédé de fabrication de la composition pharmaceutique solide selon les revendications 9 à 14, dans lequel :
- la valeur de perte au séchage (LOD) du mélange de compression de l'étape iv) se trouve dans la plage allant de 0,05% à 1,7%, de préférence de 0,1% à 0,8%,
- le volume apparent du mélange de compression de l'étape iv) se trouve dans une plage allant de 1,5 mL/g à 3,2 mL/g, de préférence dans une plage allant de 1,8 mL/g à 3,0 mL/g, idéalement est d'environ 2,5 mL/g,
- la taille de particule du mélange de compression de l'étape iv), dans laquelle au moins 40% en poids, de préférence au moins 50% en poids du mélange de compression obtenu à l'étape iv) se trouvent dans la plage allant de 125 à 500 µm mesurée par analyse granulométrique.
